# EUROPEAN PATENT SPECIFICATION

(11) **EP 1 469 886 B1**
(45) Date of publication and mention of the grant of the patent: **24.05.2006**
(21) Application number: 03702813.1
(22) Date of filing: 29.01.2003
(51) Int. Cl.: A61K 47/48

(54) **LYOPHILIZED PHARMACEUTICAL COMPOSITION OF PROPOFOL**
LYOPHILISIERTE PHARMAZEUTISCHE ZUSAMMENSETZUNG VON PROPOFOL
COMPOSITION PHARMACEUTIQUE LYOPHILISÉE DE PROPOFOLE

(30) Priority: 01.02.2002 ZA 200200929; 06.08.2002 US 401633 P
(43) Date of publication of application: 27.10.2004
(73) Proprietor: Shimoda Biotech (PTY) LTD, Port Elizabeth 6001 (ZA)
(72) Inventor: PENKLER, Lawrence, John, (ZA); DAISLEY, Barry, Paul, c/o Standard Trust, Newton Park, 6057 Port Elizabeth (ZA)
(74) Representative: Curtis, Philip Anthony
(86) International application number: PCT/IB2003/000266
(87) International publication number: WO 2003/063824

(56) References cited:
- WO-A-01/97796
- WO-A-96/32135
- WO-A-96/41646
- TRAPANI G ET AL: "Effect of 2-hydroxypropyl-[beta]-cyclodextrin on the aqueous solubility of the anaesthetic agent propofol (2,6-diisopropylphenol)" INTERNATIONAL JOURNAL OF PHARMACEUTICS 1996 NETHERLANDS, vol. 139, no. 1-2, 1996, pages 215-218, XP002252590 ISSN: 0378-5173
- TRAPANI G. ET AL JOURNAL OF PHARMACEUTICAL SCIENCES vol. 87, no. 4, April 1998 - 1998, pages 514 - 518

## Description

### BACKGROUND OF THE INVENTION

This invention relates to a pharmaceutical composition containing an inclusion complex of propofol arid a water soluble cyclodextrin, in freeze dried form.

Propofol (2,6 di-isopropylphenol) is widely used as an induction agent for anaesthesia. The high lipophilicity of the agent facilitates rapid penetration of the drug into the central nervous system thus providing rapid onset of action. Owing to the strong lipophilic nature of propofol, the commercial intravenous preparation is formulated as a lipid emulsion in soya bean oil, egg phospholipids and glycerol. Formation of the emulsion demands sophisticated industrial processes to ensure nanoparticulate dimensions of the lipid phase to enable sterilization by filtration and maintain stability.

Significant pain on injection has been associated with the emulsion formulation. [Propofol , the newest induction agent of anaesthesia; Kanto, J. H. int. J. Clin. Pharmacol. Ther. Toxicol. 1988, 26 (1) 41-57]. Propofol is currently available commercially as 20 milliliter ampoules containing 10 mg/ml propofol. Propofol is given by intravenous injection for the induction of anaesthesia by administering 40 mg every 10 seconds. Most adults will be anaesthetized by a dose of 2,0 to 2,5 mg per kg body-weight [Martindale 29, 1125]. The same propofol formulation has also been used as a retention enema for pre-operative paediatric sedation, where the contents of the ampoule are introduced rectally. The commercially available emulsion does not however have acceptable retention characteristics.

There is a need for safe and effective intravenous formulations of propofol which are industrially simple to produce and which may cause less pain on injection, and which may also be used as retention enemas.

Cyclodextrins are cyclic oligosaccharides with a cone-like shape. The interior of the cone behaves as a hydrophobic cavity whilst the exterior of the cone is hydrophilic. The former property enables cyclodextrins to form inclusion complexes with a wide variety of lipophilic molecules which "fit" into the cavity while the latter property facilitates aqueous solubility. Cyclodextrin derivatives such as 2-hydroxypropylated beta-cyclodextrins have been extensively-studied for use as parenteral drug carriers owing to their high water solubility and low toxicity [Cyclodextrins in Pharmacy. Frömming, K-H. & Szejtli, J. 1994 Kluwer Academic Publishers pp 1-44]. A particularly useful grade of 2-hydroxypropyl-beta-cyclodextrin is produced when the degree of 2-hydroxypropylation is controlled to between 3,9 and 5,1 2-hydroxypropyl substituents per beta-cyclodextrin molecule [ZA Pat. No. 84/10042 to Janssen Pharmaceutica] commercially available as Encapsin-HPB™. Clinical studies on 8 healthy volunteers showed that intravenous infusion of Encapsin-HPB at a dose of 3 g, given as a single dose, was safe and well tolerated [Junge, W. and Seiler, K.-U. Janssen Clinical Research Report July 1988].

The following prior art is known in relation to cyclodextrins and propofol.

PCT International patent publication no. WO 9317711 to Australian Commercial Research and Development Limited, Australia teaches the preparation of 6A-amino-6A-N-(4-aminobutyl)-6A-deoxy-beta-cyclodextrin derivatives and formulation of inclusion complexes of propofol or alfaxolone with the modified cyclodextrins.

The preparation and central action of propofol/hydroxypropyl-beta-cyclodextrin complexes in rabbits has been reported [Viemstein, H., Stumpf Ch., Spiegl, P. and Reiter, S. Arzneim. -Forsch. 1993, 43, 818-823]. Solid inclusion complexes of propofol/2-hydroxypropyl-beta-cyclodextrin were prepared. by dissolving the propofol in ethanol to obtain concentrations between 10 and 20 %. One part of the drug solution was added to up to three parts of 2-hydroxypropyl-beta-cyclodextrin and kneaded in a mortar until the solvent evaporated. The resulting powder was dried under vacuum to constant weight. Proof of inclusion complexation in the solid state was provided by infrared spectroscopic analysis and differential scanning calorimetry. From phase solubility studies in phosphate buffer the stability constant of the propofol/2-hydroxypropyl-beta-cyclodextrin complex with 1:1 mol/mol stoichiometry was estimated to be 2940 M⁻¹. The solid complexes were dissolved in artificial plasma, leading to a colloidal solution which was suitable for intravenous administration. Compared with commercial propofol injection no differences were observed between the two dosage forms in the onset, duration and maximal effect.

In a study to determine smooth muscle relaxant effects of propofol and ketamine in isolated guinea pig trachea, propofol was prepared as a 0,1M aqueous solution in 40% w/v 2-hydroxypropyl-beta-cyclodextrin (corresponding to 17,83mg/ml propofol in 400mg/ml 2-hydroxypropyl-beta-cyclodextrin or a mass ratio of 1:22,4 or a molar ratio of 1:2,8 mol/mol propofol to 2-hydroxypropyl-beta-cyclodextrin) and as the commercial lipid emulsion [Pedersen, C.M., Thirstup, S. and Nielsen-Kudsk, J.E. Eur. J. Pharmacol. 1993, 238, 75-80]. Propofol showed three times higher muscle relaxant activity when solubilized with 2-hydroxypropyl-beta-cyclodextrin compared with the lipid emulsion.

The interaction between propofol and HPBCD in solution as well as anaesthetic properties of a solution of propofol in HPBCD have been described (Trapani et al J Pharm Sci 1998, 87, 514-518). A freeze-dried complex was prepared by producing an equimolar solution of propofol and HPBCD (0.418g propofol, 2.35 mmol and 3.24g, 2.35 mmol HPBCD) in 10ml deionised water. The opalescent mixture was stirred at room temperature for 5 days, filtered and freeze-dried. No data for the solid complex are given.

International patent application no. PCT/GB 96/00737 to South African Druggists Limited teaches a liquid pharmaceutical composition for administration as an injection or as a retention enema containing an inclusion complex of propofol and 2-hydroxypropyl-beta-cyclodextrin, with approximately a 1:2 mol/mol stoichiometry, the composition including a co-solvent where necessary. The pharmaceutical composition is prepared by dissolving in water an amount of 2-hydroxypropyl-beta-cyclodextrin and then adding, with mixing, an amount of propofol to provide the desired molar ratio, and if necessary, adding the pharmaceutically acceptable co-solvent.

The effect of 2-hydroxypropyl-β-cyclodextrin (HPBCD) on the aqueous solubility of propofol has been reported (Trapani et al lnt. J. Pharmaceutics 1996, 139, 215-218). The authors describe two methods of preparing a "complex" between propofol and HPBCD: (i) cosolvent evaporation and (ii) freeze drying. In both cases an excess of propofol was equilibrated with a 40.5% w/v solution of HPBCD in water over 4 days under nitrogen, then dried by rotary evaporation or by freeze drying. No data is provided to prove complexation in fact took place (e.g. X-ray diffraction, infrared spectroscopy or differential scanning calorimetry). No data is provided to indicate that the complexes so prepared were pharmaceutically acceptable powder plugs, readily soluble or stable at room temperature. The product contained 17.4 mg propofol per gram.

The effect of HPBCD on the cardiovascular profile of propofol in rats has been reported (Bielsen, SJ et al Anesth Analg 1996, 82, 920-924) in which 5mg/ml propofol in a 20% w/v solution of HPBCD was administered to rats.

PCT International patent publication no. WO 01/97796 to Bharat Serums and Vaccines Limited discloses clear aqueous solutions of propofol in hydroxyl-propyl-beta-cyclodextrin (HPBCD). This reference has two serious shortcomings from a commercialization perspective:
1) the preparation is only stable at 2-8 °C (which presents logistical problems in product distribution and hospital storage); and
2) it uses 30-60% HPBCD concentrations (which has a significant impact on safety for maintenance anaesthesia and product cost).

Freeze-dried preparations of ketoprofen and methyl-beta-cyclodextrin have been described (Drug-Dev-Ind-Pharm; 1994; 20(12); 1957-1969) in which solutions of ketoprofen and dimethyl-beta-cyclodextrin were freeze-dried to determine whether an inclusion complex was formed. The products obtained were amorphous mixtures of the 2 components. X-ray diffraction, differential scanning calorimetry, NMR spectroscopy, and thermofractography gave no proof of an inclusion of ketoprofen in the cyclodextrin cavity. It was concluded that no inclusion compound is formed in freeze-dried products of ketoprofen and dimethyl-beta-cyclodextrin. However it is well established that ketoprofen does in fact form an inclusion complex with a variety of cyclodextrins, including beta-cyclodextrin, in solution with a high stability (Pharm Dev Technol 1998 Aug;3(3):307-13). It is therefore apparent that mere freeze-drying of a solution of drug and cyclodextrin may not result in a complex isolated in the solid state.

### SUMMARY OF THE INVENTION

According to a first aspect of the invention there is provided a pharmaceutical composition in freeze dried form comprising an inclusion complex of propofol and a water soluble cyclodextrin, wherein the molar ratio of propofol to cyclodextrin is 1: greater than 1.

The water soluble cyclodextrin is preferably hydroxypropyl-beta-cyclodextrin, in which case the molar ratio of propofol to cyclodextrin is preferably 1:1.5 to 1:2.

According to a second aspect of the invention there is provided a pharmaceutical composition in freeze dried form comprising an inclusion complex of propofol and a water soluble cyclodextrin, and a lyoprotectant.

The lyoprotectant may be for example glycerol, polyethyleneglycol or ethanol.

According to a third aspect of the invention there is provided a pharmaceutical composition in unit dose form, the pharmaceutical composition being freeze dried and comprising an inclusion complex of propofol and a water soluble cyclodextrin, the unit dose containing from 50 mg to 400 mg inclusive of propofol.

The pharmaceutical compositions of the invention have the advantages that the inclusion complex of propofol and the water soluble cyclodextrin is present in amorphous solid form which means that the compositions are stable. Further, the pharmaceutical compositions of the invention may easily be reconstituted by dissolution in water to provide a solution for injection.

According to a fourth aspect of the invention there is provided a method of making a pharmaceutical composition in freeze dried form comprising an inclusion complex of propofol and a water soluble cyclodextrin, including the steps of:
(a) preparing a solution of propofol and the cyclodextrin, containing greater than 70% mass to volume of the cyclodextrin; and
(b) subjecting the solution of step (a) to a rapid freezing cycle and subsequently freeze drying to complete dryness to give the freeze dried pharmaceutical composition.

Step (b) of the method of the invention preferably includes the steps of:
(i) filling the solution of step (a) into a vial;
(ii) placing the vial in a freezing chamber so that the temperature of the solution in the vial drops to below -30°C, preferably about -35°C;
(iii) evacuating the vial and increasing the temperature in the freezing chamber to between about 5°C and 15°C inclusive, preferably about 10°C;
(iv) when the product in the vial reaches the temperature of the freezing chamber, increasing the temperature in the freezing chamber to about 25°C to 35°C inclusive, preferably about 30°C and drying the product to dryness; and
(v) breaking the vacuum under dried dinitrogen and sealing the vial to give the pharmaceutical composition.

### DESCRIPTION OF EMBODIMENTS

The crux of the invention is a pharmaceutical composition in freeze dried form comprising an inclusion complex of propofol and a water soluble cyclodextrin.

In one aspect of the invention, the molar ratio of propofol to cyclodextrin in the inclusion complex is 1: greater than 1.

In a second aspect of the invention, the pharmaceutical composition includes a lyoprotectant in an amount which ensures optimal consistency of the freeze dried composition and rapid and complete dissolution of the freeze dried composition.

In a third aspect of the invention, the pharmaceutical composition is provided in unit dose form, each unit dose containing from 50 mg to 400 mg inclusive of propofol.

A general description of the various aspects of the invention now follows.
To illustrate the invention, there is described the preparation of a pharmaceutical composition, in freeze dried form, comprising an inclusion complex of propofol and 2-hydroxypropyl-beta-cyclodextrin (HPBCD).

Such pharmaceutical compositions are stable and may be rapidly dissolved for reconstitution, facilitating ease of use in a clinical setting.

A concentrated aqueous solution of HPBCD (79.1% m/v) is prepared at 30°C. The required amount of propofol is gradually added with vigorous stirring. Stirring is continued for 1 hour and the heat is removed. The clear solution is stirred until room temperature is reached.

The preferred molar stoichiometry of propofol to HPBCD is 1:1.5 to 1:2.

The average degree of substitution of the 2-hydroxypropyl-beta-cyclodextrin is preferably between 2,5 and 9,0 and more preferably between 4.6 and 5,1 2-hydroxypropyl groups per beta-cyclodextrin molecule, 2-hydroxypropyl-beta-cyclodextrin with an average degree of substitution of 4,6 has a corresponding average molecular mass of around 1400 grams per mole as determined by nuclear magnetic resonance spectrometry. The mass ratio of propofol to 2-hydroxypropyl-beta-cydodextrin may be between 1:11,78 to 1:15.7 when the average degree of substitution is 4,6 2-hydroxypropyl groups per cyclodextrin molecule.

The solution may contain other physiologically compatible compounds such as an anti-oxidant, for example acetylcysteine and/or EDTA, or sodium metabisulphite or potassium nitrate, or monothioglycerol and/or a preservative, for example benzalkonium chloride or bronopol or chlorhexidine gluconate or chlorobutanol, or a pharmaceutically acceptable lyoprotectant such as a glycerol, polyethylene glycol or ethanol.

The solution may contain a buffer such as TRIS or similar organic amine buffer to maintain the pH of the solution between 7 and 7.4.

The solution is brought to final volume and is degassed with nitrogen. The solution is sterilized by filtration and aseptically transferred into vials under a nitrogen atmosphere.

The solution is subjected to a rapid freezing cycle with chamber at less than -20°C, preferably less than -30°C and most preferably at -35°C. Once the product temperature drops below -30°C, the product is evacuated at 0.2mbar and the shelf temperature increased to 10°C. Once equilibration is reached (i.e. product temperature reaches 10°C) the shelf temperature is increased to 30°C and drying continued to complete dryness. The vacuum is broken with dried dinitrogen and the vials sealed.

After reconstitution with water for injection, the composition of the invention preferably has a concentration of propofol of 5 mg per millilitre, more preferably about 10 or 20 mg per milliter up to 30 mg per millilitre.

The composition may be formulated in unit dose form, each unit dose containing from 50 to 400 mg inclusive of propofol. The preferred unit dose contains 200 mg propofol.

The compositions of the invention may be used for the induction and maintenance of anaesthesia by intravenous injection.

The compositions are suitable for further dilution if required in conventional intravenous diluents such as water for injection or dextrose solution.

The compositions are suitable for Y-site administration in suitable solutions such as dextrose solution.

The compositions are also suitable for use as a retention enema, particularly for pre-operative paediatric sedation. The aqueous nature of the compositions has advantages over the conventional emulsion-based formula, as aqueous formulations are generally better retained than oily formulations.

In order to illustrate the advantage of the present invention over the prior art, the following parameters have been calculated based on the data provided in the prior art. The freeze-drying time is calculated on the basis of 5.1 h per ml water.

| | **mg Propofol** | **Volume H2O*** | **Freeze-drying** |
|---|---|---|---|
| **Source** | **per gram complex** | **(ml)** | **time (h)** |
| Trapani 1998 | 114 | 4.78 | 24 |
| Trapani 1996 | 17.4 | 195 | >48 |
| Invention | 78.2 | 2.9 | 14.8 |

| | | | |
|---|---|---|---|
| * normalized for content of 200mg propofol | | | |

Compositions according to the invention therefore present advantages in respect of reduced drying time and consequently are significantly more economical than those taught in the prior art.

Compositions according to the invention also have important clinical benefits over the currently available commercial fat emulsion based preparations, namely:
(i) lower potential for bacterial growth;
(ii) abolishes the hypertriglyceridemia associated with prolonged propofol infusion; and
(iii) potential to reduce pain on injection.

### EXAMPLE 1

In an aseptic processing environment 23.56 g 2-hydroxypropyl-beta-cyclodextrin D.S 4,6 (Roquette) is weighed and transferred to a sterile graduated mixing vessel. Water for injection (29 ml) is added with stirring. The mixture is warmed to 30 °C. Stirring is continued until a clear solution is obtained. Two grams of propofol (Ethyl Corporation) is gradually introduced over a 5 minute period to the concentrated 2-hydroxypropyl-beta-cyclodextrin solution with vigorous stirring. The heat is removed and stirring is continued. Once ambient temperature is reached the solution is bubbled with nitrogen for 20 minutes. The solution is passed through 0,22 micron filter into presterilized glass vials such that each vial contains the equivalent of 200mg propofol. The vials are subjected to a rapid freezing cycle with the chamber at -35°C. Once product temperature is less than -30°C, the vials are evacuated at 0.2mbar and the shelf temperature increased to 10°C. Once product temperature reaches -10°C, the shelf temperature is increased to 30°C and drying continued to complete dryness. The vacuum is broken with dried dinitrogen and the vials sealed under nitrogen. The product is a white powder plug. Reconstitution with 18.2 ml water for injection produces a clear solution in under 60 seconds. Propofol content is verified by HPLC analysis to be 10,0 ± 0,1 mg propofol per ml.

### EXAMPLE 2

### Batch Size 50 x 4 ml Vials

| | |
|---|---|
| Propofol | 2.00g |
| HPBCD | 31.40g |
| MTG | 100mg |
| Purified water | ad 200ml |

### Bulk Solution

Heat 50ml water to 80°C in a 250ml graduated conical flask on a heated magnetic stirring plate. Gradually add HPBCD with stirring until completely dissolved. Remove heat and allow to cool to room temperature. Bubble dinitrogen through solution for 20 minutes and add monothioglycerol (MTG) with stirring under dinitrogen atmosphere. Add propofol gradually via syringe with vigorous stirring under dinitrogen. Once propofol addition is complete, stir for 30 minutes under dinitrogen. Preservatives and lyoprotectants may be added to the composition at this stage. Add water to 200ml mark and continue stirring for 30 minutes. The solution is filtered with a hydrophilic PVDF 0.45 micron syringe filter. Aliquots of 4ml are pipetted into vials. The filled vials are then freeze dried as per Example 1. The vacuum is broken with dried dinitrogen and the vials sealed under nitrogen. The product is a solid white powder plug. Reconstitution with 3.4 ml water for injection produces a clear solution in under 60 seconds. Propofol content is verified by HPLC analysis to be 10,0 ± 0,1 mg propofol per ml.

## Claims

1. A pharmaceutical composition in freeze dried form comprising an inclusion complex of propofol and a water soluble cyclodextrin, wherein the molar ratio of propofol to cyclodextrin is 1: greater than 1.

2. The pharmaceutical composition according to claim 1, wherein the water soluble cyclodextrin is hydroxypropyl-beta-cyclodextrin.

3. The pharmaceutical composition according to claim 2, wherein the molar ratio of propofol to cyclodextrin is 1:1.5 to 1:2.

4. A pharmaceutical composition in freeze dried form comprising an inclusion complex of propofol and a water soluble cyclodextrin, and a lyoprotectant.

5. The pharmaceutical composition according to claim 4, wherein the lyoprotectant is a glycerol, polyethyleneglycol or ethanol.

6. A pharmaceutical composition in unit dose form, the pharmaceutical composition being freeze dried and comprising an inclusion complex of propofol and a water soluble cyclodextrin, the unit dose containing from 50 mg to 400 mg inclusive of propofol.

7. A pharmaceutical composition in unit dose form according to claim 6, the unit dose containing 200mg propofol.

8. A method of making a pharmaceutical composition in freeze dried form comprising an inclusion complex of propofol and a water soluble cyclodextrin, including the steps of:
(a) preparing a solution of propofol and the cyclodextrin, containing greater than 70% mass to volume of the cyclodextrin; and
(b) subjecting the solution of step (a) to a rapid freezing cycle and subsequently freeze drying to complete dryness to give the freeze dried pharmaceutical composition.

9. The method of claim 8, wherein step (b) of the method of the invention includes the steps of:
(i) filling the solution of step (a) into a vial;
(ii) placing the vial in a freezing chamber so that the temperature of the solution in the vial drops to below -20°C;
(iii) evacuating the vial and increasing the temperature in the freezing chamber to between about 5°C and 15°C inclusive;
(iv) when the product in the vial reaches the temperature of the freezing chamber, increasing the temperature in the freezing chamber to about 25°C to 35°C inclusive, and drying the product to dryness; and
(v) breaking the vacuum under dried dinitrogen and sealing the vial to give the pharmaceutical composition.

10. The method of claim 9, wherein, in step (ii) of step (b), the temperature of the solution in the vial drops to below -30°C.

11. The method of claim 10, wherein, in step (ii) of step (b) the temperature of the solution in the vial drops to about -35°C.

12. The method of claim 9, 10 or 11 wherein, in step (iii) of step (b), the temperature in the freezing chamber is increased to about 10°C.

13. The method of claim 9, 10, 11 or 12 wherein, in step (iv) of step (b), the temperature in the freezing chamber is increased to about 30°C.

## Patentansprüche

1. Eine pharmazeutische Zusammensetzung in gefriergetrockneter Form enthaltend einen Einschlusskomplex von Propofol und einem wasserlöslichen Cyclodextrin, wobei das molare Verhältnis von Propofol zu Cyclodextrin 1: größer als 1 ist.

2. Die pharmazeutische Zusammensetzung gemäß Anspruch 1, wobei das wasserlösliche Cyclodextrin Hydroxypropyl-beta-Cyclodextrin ist.

3. Die pharmazeutische Zusammensetzung gemäß Anspruch 2, wobei das molare Verhältnis von Propofol zu Cyclodextrin 1 : 1,5 bis 1 : 2 ist.

4. Eine pharmazeutische Zusammensetzung in gefriergetrockneter Form enthaltend einen Einschlusskomplex von Propofol und einem wasserlöslichen Cyclodextrin und einen Lyoprotektor.

5. Die pharmazeutische Zusammensetzung gemäß Anspruch 4, wobei der Lyoprotektor ein Glycerin, Polyethylenglykol oder Ethanol ist.

6. Eine pharmazeutische Zusammensetzung in Einzeldosis-Form, wobei die pharmazeutische Zusammensetzung gefriergetrocknet ist und einen Einschlusskomplex von Propofol und einem wasserlöslichen Cyclodextrin enthält, und wobei die Einzeldosis 50 mg bis 400 mg einschließlich Propofol enthält.

7. Eine pharmazeutische Zusammensetzung in Einzeldosis-Form gemäß Anspruch 6, wobei die Einzeldosis 200 mg Propofol enthält.

8. Ein Verfahren zur Herstellung einer pharmazeutischen Zusammensetzung in gefriergetrockneter Form enthaltend einen Einschlusskomplex von Propofol und einem wasserlöslichen Cyclodextrin, das folgende Schritte enthält:
a) Herstellen einer Lösung von Propofol und dem Cyclodextrin, enthaltend mehr als 70% Masse zu Volumen des Cyclodextrins; und
b) Die Lösung aus Schritt (a) einem schnellen Gefrierzyklus unterwerfen und nachfolgend bis zur vollständigen Trockene gefriertrocknen, um die gefriergetrocknete pharmazeutische Zusammenset zung zu erhalten.

9. Das Verfahren gemäß Anspruch 8, wobei Schritt (b) des erfindungsgemäßen Verfahrens folgende Schritte enthält:
(i) Abfüllen der Lösung aus Schritt (a) in eine Flasche;
(ii) Platzieren der Flasche in einer Gefrierkammer, so dass die Temperatur der Lösung in der Flasche unter -20°C fällt;
(iii) Evakuieren der Flasche und Erhöhen der Temperatur in der Gefrierkammer auf zwischen ca. 5°C und 15°C einschließlich;
(iv) wenn das Produkt in der Flasche die Temperatur der Gefrierkammer erreicht, Erhöhen der Temperatur in der Gefrierkammer auf ca. 25°C bis 35°C einschließlich und Trocknen des Produkts zur Trockene; und
(v) Aufbrechen des Vakuums unter getrocknetem Distickstoff und Versiegeln der Flasche, um die pharmazeutische Zusammensetzung zu erhalten.

10. Das Verfahren nach Anspruch 9, wobei in Schritt (ii) von Schritt (b) die Temperatur der Lösung in der Flasche auf unter -30°C abfällt.

11. Das Verfahren nach Anspruch 10, wobei in Schritt (ii) von Schritt (b) die Temperatur der Lösung in der Flasche auf ca. - 35°C abfällt.

12. Das Verfahren nach Anspruch 9, 10 oder 11, wobei in Schritt (iii) von Schritt (b) die Temperatur in der Gefrierkammer auf ca. 10°C erhöht wird.

13. Das Verfahren nach Anspruch 9, 10, 11 oder 12, wobei in Schritt (iv) von Schritt (b) die Temperatur in der Gefrierkammer auf ca. 30°C erhöht wird.

## Revendications

1. Composition pharmaceutique sous forme lyophilisée comprenant un complexe en inclusion de propofol et d'une cyclodextrine soluble dans l'eau, où le rapport molaire du propofol à la cyclodextrine est de 1:plus de 1.

2. Composition pharmaceutique selon la revendication 1, où la cyclodextrine soluble dans l'eau est l'hydroxypropyl-béta-cyclodextrine.

3. Composition pharmaceutique selon la revendication 2, où le rapport mollaire du propofol à la cyclodextrine est de 1:1,5 à 1:2.

4. Composition pharmaceutique sous forme lyophilisée comprenant un complexe en inclusion de propofol et d'une cyclodextrine soluble dans l'eau, et un lyoprotecteur.

5. Composition pharmaceutique selon la revendication 4, où le lyoprotecteur est un glycérol, un polyéthylèneglycol ou un éthanol.

6. Composition pharmaceutique sous forme de dose unitaire, la composition pharmaceutique étant lyophilisée et consistant en un complexe en inclusion de propofol et d'une cyclodextrine soluble dans l'eau, la dose unitaire contenant 50 mg à 400 mg inclus de propofol.

7. Composition pharmaceutique sous forme de dose unitaire selon la revendication 6, la dose unitaire contenant 200 mg de propofol.

8. Méthode de production d'une composition pharmaceutique sous forme lyophilisée comprenant un complexe en inclusion de propofol et d'une cyclodextrine soluble dans l'eau, comprenant les étapes de :
(a) préparer une solution de propofol et de la cyclodextrine, contenant plus de 70% en masse par rapport au volume de la cyclodextrine ; et
(b) soumettre la solution de l'étape (a) à un cycle de congélation rapide et subséquemment une lyophilisation pour obtenir la siccité pour donner la composition pharmaceutique lyophilisée.

9. Méthode de la revendication 8 où l'étape (b) de la méthode de l'invention comprend les étapes de :
(i) introduire la solution de l'étape (a) dans une fiole ;
(ii) placer la fiole dans une chambre de congélation de façon que la température de la solution dans la fiole baisse à moins de -20°C.
(iii) évacuer la fiole et augmenter la température dans la chambre de congélation à une valeur comprise entre environ 5°C et 150°C inclus ;
(iv) quand le produit dans la fiole atteint la température de la chambre de congélation, augmenter la température dans la chambre de congélation à environ 25°C à 35°C inclus, et sécher le produit à siccité ; et
(v) rompre le vide sous diazote séché et sceller la fiole pour donner la composition pharmaceutique.

10. Méthode de la revendication 9, où à l'étape (ii) de l'étape (b), la température de la solution dans la fiole baisse à moins de -30°C.

11. Méthode de la revendication 10, où à l'étape (ii) de l'étape (b), la température de la solution dans la fiole baisse à environ -35°C.

12. Méthode de la revendication 9, 10 ou 11 où, à l'étape (iii) de l'étape (b), la température dans la chambre de congélation est augmentée à environ 10°C.

13. Méthode de la revendication 9, 10, 11 ou 12 où, à l'étape (iv) de l'étape (b), la température dans la chambre de congélation est augmentée à environ 30°C.
